Europäisches Patentamt

(19) European Patent Office

Office européen des brevets

(11) Publication number: **0 125 036**
**B1**

(12) EUROPEAN PATENT SPECIFICATION

(45) Date of publication of patent specification: **01.07.87**

(51) Int. Cl.⁴: **A 61 K 6/08**

(21) Application number: **84302436.5**

(22) Date of filing: **10.04.84**

(54) **Opaque dental porcelain and method of using the same.**

(30) Priority: **11.04.83 US 484015**

(43) Date of publication of application:
**14.11.84 Bulletin 84/46**

(45) Publication of the grant of the patent:
**01.07.87 Bulletin 87/27**

(84) Designated Contracting States:
**AT BE CH DE FR GB LI NL SE**

(56) References cited:
**US-A-4 107 348**
**US-A-4 110 184**
**US-A-4 264 640**

(73) Proprietor: **JOHNSON & JOHNSON DENTAL PRODUCTS COMPANY**
**20 Lake Drive CN 7060**
**East Windsor New Jersey 08520 (US)**

(72) Inventor: **Martin, Brian**
**290 North Post Road**
**Princeton Junction, NJ 08550 (US)**
Inventor: **Panzera, Carlino**
**7 Huntsman Lane**
**Belle Mead, NJ 08502 (US)**

(74) Representative: **Jones, Alan John et al**
**CARPMAELS & RANSFORD 43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 125 036

## Description

The invention relates to an opaque dental porcelain paste composition that can be applied to a base, and which does not have to be fired before the body porcelain layers are applied on top of the opaque porcelain layer. The invention also relates to a method of employing the opaque dental porcelain paste.

### Background of the invention

Today, most dental crowns and bridges are produced by a process which employs metal alloys and dental porcelains. The alloy frames, usually referred to as "copings", are prepared by the lost wax investment casting process. A variety of dental porcelains are then applied to the coping. The porcelain simulates the natural tooth both functionally and aesthetically, and is ordinarily applied in two stages. First, an opaque porcelain layer (which is used to mask the coping) is applied as an aqueous slurry to the surface of the metal coping, and this opaque layer is then dried and fired. Additional opaque porcelain is sometimes applied, dried, and fired if the initial application is either non-uniform or does not completely mask the alloy substructure. Once the opaque porcelain has been satisfactorily applied, the body porcelain layers, usually a gingival and an incisal porcelain, are applied as aqueous slurries and then dried and fired. A minimum of two firings is required; this includes a minimum of one firing for the opaque porcelain layer and a minimum of one firing for the gingival and incisal porcelain layers.

This invention provides an opaque dental porcelain paste composition that does not have to be fired prior to the application of subsequent porcelain layers. As a result, both time and energy can be saved by practicing this invention since at least one firing procedure can be eliminated during the process for producing a dental crown and bridge.

### Brief summary of the invention

The opaque dental porcelain paste composition of the invention comprises a dental porcelain powder opacifying pigment, and an aqueous colloidal urethane polymer dispersion, wherein said urethane polymer contains carboxyl groups and wherein the carboxyl groups are neutralized with alkali metal, ammonia or amine. Preferably, the opaque porcelain formulation has the same firing or maturing temperature as the subsequent body porcelain layers. In an important aspect of the invention, there is provided a package comprising the above-described opaque dental porcelain paste composition in a suitable container.

The opaque dental porcelain paste composition of the invention, when applied as a film or layer on a metal coping, dries rapidly (i.e. in about 20 minutes or less) to a water-resistant film. The urethane binder in the composition burns off when the dental prothesis is fired without causing harmful voids, bubbles, cracks, or other similar defects. The paste composition itself has an acceptable shelf life, so that it can be pre-mixed by the manufacturer. This is advantageous for several reasons. First, the ingredients can be mixed in the optimum proportions. Second, the pre-mixed composition can be packaged in a dispenser/container designed to dispense only the quantity desired by the dental technician. Substantial savings in material can therefore be realized by the user, because the user can readily dispense only the required amount.

### The prior art

Infante, in U.S. Patent Nos. 4,046,732 and 4,264,640, describes earlier approaches toward the solution of the problem to which the subject invention relates. Infante describes dispersants for incorporation in both the opaque porcelain layer and the subsequent body porcelain layers in dental prostheses. These dispersants are aqueous solutions of various acrylic polymers. It is apparently an essential feature of Infante's invention that both the opaque porcelain layer and the body porcelain layers employ the dispersants. This can be disadvantageous because the body porcelain layer(s) are usually applied in much thicker layers than the opaque porcelain layer, and are therefore more apt to bubble or form harmful voids or cracks when they contain ingredients that must be burned off.

Hirschhorn, in U.S. Patent No. 4,107,348, describes a resinous binder solution in water or water-alcohol, for use with the opaque porcelain layer in making a dental prosthesis.

### Detailed description of the invention

The opaque porcelain powders that are used in the invention broadly constitute a known class of compositions. They are typically composed of silica, alumina, alkali metal oxides, alkaline earth metal oxides, boron oxide, opacifiers (which, in some cases, may also serve as white pigments) such as tin (stannic) oxide, titanium dioxide, zirconium silicate, and calcium silicate, and yellow, pink, and brown pigments such as the oxides and salts of vanadium, chromium, manganese, and other ceramic pigments and coloring agents whose nature and mode of use are known in the art. The nature, specific compositions, and methods of preparation of such opaque dental porcelains are known, and need not be repeated here.

In one preferred aspect of the invention, the opaque porcelain is selected to have a firing or maturing temperature of 982±5.5°C (1800±10°F), in order to match the firing temperatures of the body porcelain layers (gingival and incisal) that are to be applied on top of the opaque porcelain layer. The examples, below, give a specific illustration of an opaque porcelain formulation that meets this requirement.

2

The principal novelty of this invention resides in the use of the aqueous urethane polymer dispersion in conjunction with the opaque porcelain powder. The resulting mixture, which is a paste (often called a "slurry" in the dental art), in a preferred aspect is relatively stable and can therefore be pre-mixed by the manufacturer in the correct proportions, and can be packaged in convenient ready-to-use container/applicators such as flexible tubes and syringes.

Aqueous colloidal dispersions of urethane polymers are commercially available materials. Therefore, their nature and production are known in the art. For instance, they are described in the following U.S. Patents.

Milligan et al., No. 3,412,054
Witt et al., No. 3,870,684
Hirooka et al., No. 3,983,058
Scriven et al., No. 4,066,591
Knachkamp et al., No. 4,172,191
Williams et al., No. 4,329,490.

(These patents are merely illustrative of many that teach the preparation of the aqueous colloidal urethane dispersions).

The urethane polymers used to make the aqueous colloidal dispersions are substantially linear polymers that comprise the reaction products of polyether and/or polyester diols with organic diisocyanates. Useful polyether diols include polypropylene glycols, mixed polyethylene-polypropylene glycols, polytetramethylene ether glycols, and similar known materials. Useful polyesters include hydroxyl-terminated poly(ethylene adipate) and poly(epsilon-caprolactone). Useful organic diisocyanates include aromatic diisocyanates such as tolylene diisocyanate and diphenylmethane diisocyanate ("MDI") and aliphatic diisocyanates such as isophorone diisocyanate and bis(4-isocyanatocyclohexyl)methane.

The urethane polymers are produced by reacting the diol with diisocyanate in appropriate proportions. If desired the polyether and/or polyester diol may be reacted with a stoichiometric excess of organic diisocyanate to form an isocyanato-terminated prepolymer, which is then reacted with a difunctional chain extender. Difunctional chain extenders include diols such as ethylene glycol, diethylene glycol and 1,4-butane diol, and diamines such as ethylene diamine.

The ability of the urethane polymer to form stable aqueous colloidal dispersions is enhanced by incorporating hydrophilic groups in the polymer chains. The carboxyl groups, which, when neutralized as with alkali metal, ammonia, or an amine, form such hydrophilic groups and thereby enhance the affinity of the polymer for water. Such carboxyl group functionality may be incorporated in the polymer chain, for instance, by using as the diol extender a dihydroxy-substituted carboxylic acid such as 2,2-bis(hydroxymethyl)propionic acid.

It has been found that both (a) the addition to the aqueous phase of small amounts of anions that form salts with calcium or barium that have a solubility in water below 0.5 weight per cent, and (b) the maintenance of a slightly alkaline pH, e.g. 8 to 9, enhance the stability of the paste. Such anions are illustrated by oxalate, phosphate, silicate, borate, and tartrate. The alkaline pH helps to ensure that the urethane polymer will maintain its affinity for water and will therefore not tend to coagulate. The reason for the said anion's contributing to stability is less certain; it is probable that these anions serve to keep the aqueous phase substantially free of calcium and/or barium cations (and possibly other polyvalent metal cations) by immediately precipitating such cations as they are leached out of the porcelain powder. (Alkaline pH also helps here because the tendency of calcium and barium to leach out of the porcelain is less under alkaline conditions than under acid conditions). Calcium and/or barium cations, even in very small quantities, could displace the solubilizing cation associated with the pendant carboxyl groups of the urethane polymer, and thereby tend to coagulate the urethane polymer.

Other ingredients that can be employed in the paste include dispersants, suspending agents, polar organic solvents, anti-foam agents, and biocides. Dispersants, which are employed to deter particle agglomeration, are illustrated by alkali metal polycarboxylate, sodium silicate, sodium polyphosphate and lecithin. Suspending agents are used to deter particles in the paste from settling. Illustrations include water-soluble high polymers such as alkali metal polycarboxylates, colloidal inorganic powders such as silica, clay, magnesium aluminum silicate, and magnesium silicate. (Some materials can act as both dispersants and suspending agents). The approximate proportions in which all these materials are employed are illustrated in the Examples.

Example 1
Preparation of opaque porcelain
In the preparation of the opaque porcelain, a potash feldspar and two silicate glasses are employed. These materials have the following compositions (in weight per cent):

### TABLE I

| Ingredient | Potash feldspar | Silicate glass A | Silicate glass B |
|---|---|---|---|
| $SiO_2$ | 65.5 | 73.6 | 68 |
| $Al_2O_3$ | 18.6 | 1.3 | 3 |
| CaO | 0.07 | 5 | 5 |
| BaO | 0.01 | Trace | 2 |
| $Na_2O$ | 2.4 | 15.2 | 15 |
| $K_2O$ | 13.5 | 1.3 | 1 |
| $B_2O_3$ | — | — | 2 |
| MgO | — | 3 | 4 |

The opaque porcelain is composed of a blend of four components, as follows:

### TABLE II

| | | Weight per cent |
|---|---|---|
| 1. | Component A | 60 |
| 2. | Component B | 10 |
| 3. | Silicate glass B | 10 |
| 4. | Tin oxide (Harshaw® 115) | 20 |

Component A is prepared from a mixture of 94 weight per cent potash feldspar and 6 weight per cent lithium carbonate, and Component B is prepared from a 50/50 (weight) mixture of the potash feldspar and silicate glass A. Components A and B are prepared as follows:

The raw materials are crushed to a fine powder, blended, ball milled for two hours (to pass through a 0.074 mm (200 mesh U.S.) screen), and then transferred to a dense alumina crucible. The charges are fired to 1232°C (2250°F.) for 4 hours, quenched in water, crushed, and then ball milled to form a powder fine enough to pass through a 0.091 mm (165 mesh U.S.) nylon screen.

Silicate glass B is also ball milled to pass through a 0.091 mm (165 mesh U.S.) nylon screen.

The four components are then blended to form an opaque porcelain having the following overall calculated composition:

### TABLE III

| Ingredient | Weight per cent |
|---|---|
| $SiO_2$ | 52.5 |
| $Al_2O_3$ | 12.1 |
| CaO | 0.8 |
| BaO | 0.2 |
| MgO | 0.6 |
| $Na_2O$ | 3.6 |
| $K_2O$ | 8.6 |
| $Li_2O$ | 1.0 |
| $B_2O_3$ | 0.2 |
| $SnO_2$ | 20.0 |

The foregoing white opaque porcelain formulation is simply illustrative of those that can be used in the invention. It is formulated to have a coefficient of thermal expansion of about 14 to $15 \times 10^{-6}$ cm/cm/°C (so that when pigments are added, in amounts of about 5 to 15 weight per cent, the coefficient of thermal expansion will be about 13 to $14 \times 10^{-6}$ cm/cm/°C to match dental alloy), and a firing or maturing temperature of about 982°C (1800°F.) (to match the firing temperatures of the particular body porcelain layers used). If different coefficients of thermal expansion and/or maturing temperatures are desired, it is

4

**0 125 036**

within the skill of the art, having knowledge of the teachings herein, to modify the formulation so as to obtain the desired properties. It is also relevant to mention that the use of a different grade of tin oxide than Harshaw® 115 might necessitate a change in the proportions of the ingredients in the formulation. For instance, when Transelco® 304 tin oxide is used, the following formulation has the desired coefficient of thermal expansion and maturing temperature:

| Component A | 65% |
|---|---|
| Component B | 1.5% |
| Silicate Glass B | 16.5% |
| Tin oxide (Transelco®) | 17% |

Preparation of paste

A paste is made from the following ingredients:

TABLE IV

| Component | Parts by weight |
|---|---|
| Water | 148 |
| Diammonium Phosphate | 2.8 |
| Colloidal Silica[1] | 3.5 |
| 30% aqueous ammonia, to pH 8.5 | ≈0.1—0.2 |
| Isopropyl alcohol | 10.5 |
| Water soluble thickener[2] | 1.26 |
| Opaque porcelain powder | 630 |
| Anti-foam agent[3] | 0.91 |
| Aqueous colloidal urethane polymer[4] | 51.8 |

[1] Cab-O-Sil® M-5. The colloidal silica can be replaced with or supplemented by 1 to 2 parts of "Bentone® LT", a colloidal clay.

[2] CPE-15 An acrylic polymer containing carboxyl functionality; supplied by Rohm & Haas.

[3] "Foamkill"® 639AA (Crucible Chemical Company)

[4] "Neorez"® R940-A 31 per cent (by weight) solids aqueous colloidal dispersion of a polyether/tolylene diisocyanate urethane polymer containing carboxyl functionality which is neutralized with triethylamine. The dispersion contains a small amount of methyl ethyl ketone and N-methylpyrrolidone. It has a pH of 8.3 and a viscosity of 70 mPas. It is supplied commercially by Polyvinyl Chemical Industries of Wilmington, Massachusetts.

The paste is made by the following procedure:

A bowl of a Hobart mixer is charged with half of the water in the formulation, the diammonium phosphate, and the colloidal silica, and the pH is adjusted to 8.5 by addition of aqueous ammonia. This mixture is mixed for half an hour until it forms a gel. While this gel is being formed, the water soluble thickener is added to the balance of the water, and a biocide (1,2-dibromo-2,4-dicyanobutane), in an amount of about 0.05 weight per cent of the liquid components of the formulation, is dissolved in the isopropyl alcohol. After the silica has formed a gel, the aqueous solution of water soluble thickener and the biocide in isopropyl alcohol are added to the gel in the bowl of the Hobart mixer. With stirring, the porcelain powder is gradually added to the bowl, followed by the aqueous colloidal dispersion of urethane polymer and the anti-foam agent. The mixture is mixed for one hour in the Hobart mixer, and the viscosity is adjusted with water to a viscosity of 16,000 mPas, measured on a Brookfield viscometer, model RVT, using spindle No. 6 at a speed of 50 rpm.

The above-described paste is coated on metallic coupons made of dental alloy and on dental copings, in coatings of about 0.20 to 0.25 mm (8—10 mils) thickness. The coatings are brushed on the coping or the coupon, and are then air dried for 20 minutes. A layer of body porcelain is then coated on top of the layer of opaque porcelain. For the coupons, the body porcelain layer is about 2 millimeters thick. On the coping, it is shaped to represent a dental crown, so that the thickness changes depending upon where it is on the crown. The coupons and copings are then fired using the body porcelain fusion cycle, which is the following:

The coupons and copings are placed in a vacuum oven at 649°C (1200°F), and the oven is heated at a heat-up rate of 50—56°C (90°—100°F), per minute to 927°C (1700°F). Air is then let in the oven, and it is heated at the same heat-up rate to 982°C (1800°F). When the oven reaches 982°C (1800°F), the copings and coupons are removed and are cooled in ambient air. In all cases, the samples show excellent adhesion of the porcelain to the metal, with no bubbling or cracking or any other defects apparent. The color is excellent, and the aesthetics of the porcelain layers are excellent.

5

Examples 2—4 and Control Example 1
Three different pastes were made from the formulations displayed in Table V, below:

TABLE V

| Example | 2 | 3 | 4 |
|---|---|---|---|
| Water | 17 | 17 | 17 |
| 35% phosphoric acid | 0.85 | — | — |
| Oxalic acid | — | 0.3 | — |
| CPE 15 | 0.18 | 0.18 | 0.18 |
| 30% ammonia solution | 1.35 | 1.35 | 1.35 |
| R940 Urethane Dispersion | 7.38 | 7.38 | 7.38 |
| Porcelain powder | 90 | 90 | 90 |
| "Foamkill"® 639AA | 0.13 | 0.13 | 0.13 |

These paste formulations were evaluated for water resistance by the following procedure. The formulation is brushed onto a metal panel in a thickness of about 0.20 to 0.25 mm (8—10 mils). It is either allowed to air dry for 20 minutes in ambient air, or to air dry for 20 minutes in ambient air with an additional 5 minutes at 50°C. After this, the coatings are evaluated for water resistance by the following procedure: Water is poured onto the coating, and a soft brush is brushed across the coating and back again. This constitutes one double rub. Light pressure is applied while brushing. The end of the test is reached when metal shows through the coating, although the test is stopped at 50 double rubs. In Table VI below, the results of the test are shown for these three paste formulations, compared with a commercial acrylic formulation of the type described in the two Infante patents that are cited above.

TABLE VI

| Example | 2 | 3 | 4 | Commercial acrylic formulation |
|---|---|---|---|---|
| After 20 minute air dry | >50 | 40 | 20 | 3 |
| After 20-minute air dry+5 mins. at 50°C.[5] | >50 | >50 | 18 | 10 |

[5] The five-minute bake at 50°C was used because the instructions that accompany the commercial acrylic formulation suggest such a low temperature bake. The low temperature bake is not needed with the compositions of this invention.

As these results demonstrate, the paste formulation of this invention has significantly superior water resistance after drying than does the commercial acrylic formulation.

**Claims**

1. A paste suitable for applying to a dental coping as an opaque porcelain layer, which layer does not need to be fired prior to addition of a body porcelain layer, which paste comprises an opaque porcelain powder and an aqueous colloidal dispersion of a urethane polymer, wherein said urethane polymer contains carboxyl groups, and wherein the carboxyl groups are neutralized with alkali metal, ammonia, or amine.

2. The paste of claim 1 wherein the aqueous phase contains an anion that forms a salt with calcium or barium that has a solubility in water below 0.5 weight percent.

3. The paste of claim 2 wherein said anion is phosphate, oxalate, or a mixture thereof.

4. The paste of any one of claims 1 to 3 wherein said paste contains a suspending agent and a dispersant.

5. A package comprising the paste of any one of claims 1 to 4 in a suitable applicator/container.

6. A method of producing a dental prosthesis which comprises applying a thin layer of the paste of any

6

one of claims 1 to 4 to a metal coping, drying said layer, then applying at least one layer of body porcelain paste on top of said thin layer, drying the body porcelain paste, and then baking the coping with the two layers to the firing temperature of said layers.

7. A method of producing a paste according to claim 1, comprising mixing an opaque porcelain powder with an aqueous colloidal dispersion of a urethane polymer wherein said urethane polymer contains carboxyl groups, and wherein the carboxyl groups are neutralized with alkali metal, ammonia or amine.

**Patentansprüche**

1. Zum Aufbringen auf ein Dentalkäppchen als eine opake Porzellanschicht geeignete Paste, welche Schicht nicht vor Zugabe einer Körperporzellenschicht gebrannt werden muß, welche Paste ein opakes Porzellanpulver und eine wässerige kolloidale Dispersion eines Urethanpolymers umfaßt, worin das Urethanpolymer Carboxylgruppen aufweist und worin die Carboxylgruppen mit Alkalimetall, Ammoniak oder Amin neutralisiert sind.

2. Paste nach Anspruch 1, worin die wässerige Phase ein Anion enthält, das mit Calcium oder Barium ein Salz mit einer Löslichkeit in Wasser von unter 0,5 Gew.-% ausbildet.

3. Paste nach Anspruch 2, worin das Anion Phosphat, Oxalat oder ein Gemisch hievon ist.

4. Paste nach einem der Ansprüche 1 bis 3, worin die Paste ein Suspendiermittel und ein Dispergiermittel enthält.

5. Die Paste nach einem der Ansprüche 1 bis 4 enthaltende Verpackung in einem geeigneten Applikator/Behälter.

6. Verfahren zur Ausbildung einer Dentalprothese, welches ein Aufbringen einer dünnen Schicht der Paste nach einem der Ansprüche 1 bis 4 auf ein Metallkäppchen, ein Trocknen dieser Schicht, hierauf ein Aufbringen wenigstens einer Schicht aus Körperporzellanpaste auf diese dünne Schicht, ein Trocknen dieser Körperporzellanpaste und ein anschließendes Erwärmen des Käppchens mit den beiden Schichten auf die Einbrenntemperatur dieser Schichten umfaßt.

7. Verfahren zur Herstellung einer Paste nach Anspruch 1, welches ein Vermischen eines opaken Porzellanpulvers mit einer wässerigen kolloidalen Dispersion eines Urethanpolymers, worin das Urethanpolymer Carboxylgruppen enthält und worin die Carboxylgruppen mit Alkalimetall, Ammoniak oder Amin neutralisiert sind, umfaßt.

**Revendications**

1. Pâte qui convient pour l'application sur une coiffe dentaire à titre de couche de porcelaine opaque, cette couche n'ayant pas besoin d'être cuite avant l'addition d'une couche de porcelaine de corps de dent, cette pâte comprenant une poudre de porcelaine opaque et une dispersion colloïdale aqueuse d'un polymère d'uréthane, dans laquelle ledit polymère d'uréthane contient des groupes carboxyle et où les groupes carboxyle sont neutralisés avec un métal alcalin, de l'ammoniaque ou une amine.

2. Pâte selon la revendication 1, dans laquelle la phase aqueuse contient un anion qui forme un sel avec le calcium ou le baryum qui a une solubilité dans l'eau inférieure à 0,5 pour cent en poids.

3. Pâte selon la revendication 2, dans laquelle ledit anion est le phosphate, l'oxalate ou un de leurs mélanges.

4. Pâte selon l'une quelconque des revendications 1 à 3, dans laquelle ladite pâte contient un agent de suspension et un dispersant.

5. Emballage comprenant la pâte selon l'une quelconque des revendications 1 à 4, dans un applicateur/récipient de contenance convenable.

6. Procédé pour produire une prothèse dentaire qui comporte l'application d'une couche mince de la pâte selon l'une quelconque des revendications 1 à 4, sur une coiffe métallique, le séchage de ladite couche, puis l'application d'au moins une couche de pâte de porcelaine de corps de dent au-dessus de ladite couche mince, le séchage de la pâte de porcelaine de corps de dent et ensuite la cuisson de la coiffe avec les deux couches à la température de cuisson desdites couches.

7. Procédé pour produire une pâte selon la revendication 1, comprenant le mélange d'une poudre de porcelaine opaque avec une dispersion colloïdale aqueuse d'une polymère d'uréthane, dans laquelle ledit polymère d'uréthane contient des groupes carboxyle et où les groupes carboxyle sont neutralisés avec un métal alcalin, de l'ammoniaque ou une amine.